(19) Europäisches Patentamt
European Patent Office
Office européen des brevets

(11) Veröffentlichungsnummer: **0 144 594 B1**

(12) # EUROPÄISCHE PATENTSCHRIFT

(45) Veröffentlichungstag der Patentschrift: **07.10.87**

(51) Int. Cl.⁴: **C 07 D 491/052**, A 61 K 31/505

(21) Anmeldenummer: **84111732.8**

(22) Anmeldetag: **01.10.84**

(54) **5H-[1]Benzopyrano[2,3-d]pyrimidin-Derivate, Verfahren zu deren Herstellung, sowie diese enthaltende Arzneimittel zur Bekämpfung von gastralen und duodenalen Schleimhautläsionen.**

(30) Priorität: **30.09.83 DE 3335472**

(43) Veröffentlichungstag der Anmeldung: **19.06.85 Patentblatt 85/25**

(45) Bekanntmachung des Hinweises auf die Patenterteilung: **07.10.87 Patentblatt 87/41**

(84) Benannte Vertragsstaaten: **AT BE CH DE FR GB IT LI LU NL SE**

(56) Entgegenhaltungen:
**EP - A - 0 029 934**
**EP - B - 0 008 277**

(73) Patentinhaber: **GÖDECKE AKTIENGESELLSCHAFT, Salzufer 16, D-1000 Berlin 10 (DE)**

(72) Erfinder: **Satzinger, Gerhard, Dr., Im Mattenbühl 7, D-7809 Denzlingen (DE)**
Erfinder: **Barth, Hubert, Dr., Rosenweg 60, D-7830 Emmendingen (DE)**
Erfinder: **Hartenstein, Johannes, Dr., Fohrenbühl 23, D-7801 Stegen-Wittental (DE)**
Erfinder: **Herrmann, Manfred, Dr., Wolfweg 25, D-7811 St. Peter (DE)**
Erfinder: **Fritschi, Edgar, Dr., Am Scheuerwald 2, D-7811 St. Peter (DE)**
Erfinder: **Schütt, Ilse-Dore, Dr., Lemeneckstrasse 8b, D-7804 Glottertal (DE)**

EP 0 144 594 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind 5H-[1]Benzopyrano-[2,3-d]pyrimidin-Derivate der allgemeinen Formel I

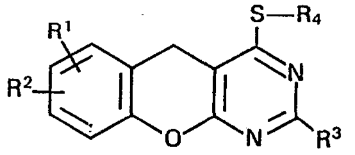

(I),

in welcher $R^1$ und $R^2$, die gleich oder verschieden sein können, ein Wasserstoff- oder Halogenatom, eine Hydroxylgruppe oder eine geradkettige oder verzweigte Alkoxygruppe mit bis zu 4 Kohlenstoffatomen, oder gemeinsam eine Alkylenoxy- oder eine Alkylendioxygruppe mit 1 bis 3 Kohlenstoffatomen oder einen ankondensierten Benzolring, $R^3$ einen unsubstituierten oder durch bis zu 3 Substituenten aus der Gruppe Halogenatome, Dialkylaminogruppen mit bis zu 4 Kohlenstoffatomen, Alkyl- oder Alkoxygruppen mit bis zu 4 Kohlenstoffatomen sowie bis zu 2 Alkylendioxygruppen mit bis zu 2 Kohlenstoffatomen substituierten Phenylrest, $R^4$ eine geradkettige oder verzweigte Alkylgruppe mit 1-8 Kohlenstoffatomen oder den Rest

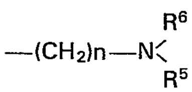

worin $R^5$ und $R^6$, die gleich oder verschieden sein können, ein Wasserstoffatom, eine geradkettige oder verzweigte Alkylgruppe mit bis zu 6 Kohlenstoffatomen oder gemeinsam mit dem Stickstoffatom eine Pyrrolidino-, Piperidino-, Morpholino- oder Piperazinogruppe bilden und n die Zahlen 2 bis 6 bedeuten,
sowie deren pharmakologisch veträgliche Salze mit organischen oder anorganischen Säuren.

Als aromatischer Ring $R^1$ und $R^2$ bevorzugt ist der unsubstituierte Phenylring in 6,7-Position. Als Halogenatome kommen Fluor, Chlor, Brom- und Jodatome in Frage. Bevorzugt ist das Bromatom. Als Alkylenoxygruppe kommt vor allem die einen Oxolenring bildende Ethylenoxygruppe in Frage. Als Alkylendioxygruppe ist der Methylendioxyrest bevorzugt, der einen 1,3-Dioxolenring bildet.

Als Substituenten des Phenylrests $R^3$ kommen bis zu 3 Substituenten aus der Gruppe Halogenatome, Dialkylaminogruppen mit bis zu 4 Kohlenstoffatomen, oder Alkyl- oder Alkoxygruppen mit bis zu 4 Kohlenstoffatomen, sowie bis zu 2 Alkylendioxygruppen mit bis zu 2 Kohlenstoffatomen in Frage.

Die Reste $R^5$ und $R^6$ bilden gemeinsam mit dem Stickstoffatom, an das sie gebunden sind, eine Pyrrolidino-, Piperidino-, Morpholino- oder Piperazinogruppe.

Bevorzugt sind Verbindungen der allgemeinen Formel I, in welcher

$R^1$ und $R^2$ gleich oder verschieden sind und ein Wasserstoff- oder Bromatom, eine Hydroxyl-, Methoxy-, oder Ethoxygruppe oder gemeinsam einen unsubstituierten, in 6,7-Position ankondensierten Benzolring,

$R^3$ einen unsubstituierten oder durch ein Halogenatom, eine Dimethylamino-, eine Methyl-, eine Methylendioxygruppe oder einen durch bis zu 3 Methoxygruppen substituierten Phenylrest,

$R^4$ eine Alkylgruppe mit bis zu 6 Kohlenstoffatomen,

$R^5$ und $R^6$, die gleich oder verschieden sein können, ein Wasserstoffatom, einen Methyl- oder Ethylrest, oder zusammen mit dem Stickstoffatom, an das sie gebunden sind, eine Pyrrolidino-, Piperidino-, Morpholino- oder Piperazinogruppe und

n die Zahlen 2 oder 3 bedeuten,
sowie deren pharmakologisch verträgliche Salze.

Besonders bevorzugt sind Verbindungen der allgemeinen Formel I, in welcher die Reste $R^1$ und $R^2$ gleich oder verschieden sind und ein Wasserstoff- oder Bromatom oder eine Methoxygruppe, $R^3$ einen Phenyl-, 4-Chlorphenyl-, 4-Dimethylaminophenyl-, 4-Methoxyphenyl-, 4-Hydroxyphenyl-, 4-Methylphenyl-, 3,4-Methylendioxyphenylrest, $R^4$ eine n-Hexylgruppe, $R^5$ und $R^6$ jeweils eine Methylgruppe darstellen und n die Zahlen 2 oder 3 bedeutet, sowie deren pharmakologisch veträglichen Salze.

Ein weiterer Gegenstand der Erfindung ist ein chemisch eigenartiges Verfahren zur Herstellung von Verbindungen der allgemeinen Formel I, welches dadurch gekennzeichnet ist, dass man entweder

a) eine Verbindung der allgemeinen Formel II,

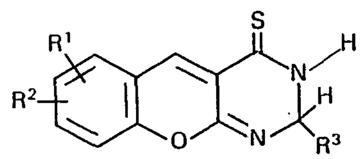

(II),

in welcher die Reste $R^1$, $R^2$ und $R^3$ die obengenannte Bedeutung haben,
in einem organischen Lösungsmittel mit einer Base tautomerisiert oder in dem man

b) eine Verbindung der allgemeinen Formel III

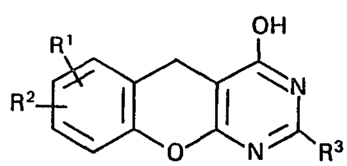

(III),

in welcher $R^1$, und $R^2$ und $R^3$ die obengenannte Bedeutung haben,
entweder

1) mit Phosphorpentasulfid in Pyridin umsetzt und anschliessend die gemäss Verfahren a) oder b) erhaltene Verbindung der allgemeinen Formel IV

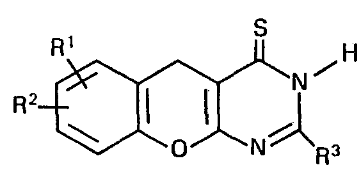

(IV),

in welcher die Reste $R^1$, $R^2$ und $R^3$ die obengenannte Bedeutung haben,
gegebenenfalls ohne Isolierung, mit einem Alkylierungsmittel der allgemeinen Formel V

$$X—R^4 \qquad (V),$$

in welcher $R^4$ die obengenannte Bedeutung hat und X eine reaktive Estergruppe bedeutet,
umsetzt oder

2) mit anorganischen Säurehalogeniden, bevorzugt mit Phosphoroxychlorid/Phosphorpentachlorid zu Verbindungen der allgemeinen Formel VI

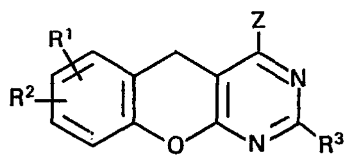

(VI),

in welcher die Reste $R^1$, $R^2$ und $R^3$ die oben genannte Bedeutung haben und Z für ein Chlor- bzw. Bromatom steht,
umsetzt und diese mit einem Thioalkohol der allgemeinen Formel IIIb

$$HS—R^4 \qquad (IIIb)$$

in welcher $R^4$ die oben genannte Bedeutung hat, zur Reaktion bringt und die so erhaltenen Basen der allgemeinen Formel I gewünschtenfalls anschliessend in an sich bekannter Weise mit organischen oder anorganischen Säuren in deren pharmakologisch unbedenkliche Salze überführt.

Bei der Umsetzung von Verbindungen der allgemeinen Formel II mit Basen [Reaktion a)] werden bevorzugt polare Lösungsmittel verwendet. Hier eignen sich besonders niedere Alkohole, wie z.B. Methanol, Ethanol oder n-Butanol.

Bei erhöhter Temperatur, bevorzugt bei der Rückflusstemperatur des Lösungsmittels beträgt die Reaktionszeit in der Regel zwischen etwa 5 und 30 Minuten.

Als Basen werden bevorzugt Kaliumcarbonat, Kaliumhydroxid, Natriummethanolat und Natriumethanolat eingesetzt.

Der Reaktionsverlauf kann gut an der deutlich sichtbaren Aufhellung der anfangs meist tief gelb bis orange gefärbten Reaktionsmischung verfolgt werden.

Die Ausgangsverbindungen der allgemeinen Formel II sind ebenfalls neue Verbindungen und werden durch Umsetzung der aus der DE-OS 2 801 353 bekannten Verbindungen der allgemeinen Formel VI

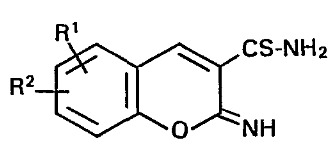

(VI),

in welcher $R^1$ und $R^2$ die obengenannte Bedeutung haben,

mit einem aromatischen Aldehyd der allgemeinen Formel VII

$$R^3\text{-CHO} \qquad (VII),$$

in welcher $R^3$ die obengenannte Bedeutung hat, in Gegenwart einer katalytischen Menge einer Base, bevorzugt bei Rückflusstemperatur erhalten. Als Basen haben sich hierfür besonders Piperidin und Triethylamin bewährt. Das bei der Reaktion entstehende Wasser entfernt man am einfachsten azeotrop, indem man ein nicht mit Wasser mischbares Lösungsmittel, wie z.B. Methylenchlorid, Chloroform, Benzol oder Toluol als Schlepper zugibt.

Die Reaktionszeit beträgt in der Regel zwischen 6 und 20 Stunden.

Die meist gelb bis orange gefärbten Verbindungen der allgemeinen Formel II fallen im Verlauf der Reaktion meist als schwerlösliche Niederschläge aus und können durch Absaugen, Auswaschen und Trocknen isoliert werden, oder aber sie werden nach Entfernen des Lösungsmittels aus dem Rückstand erhalten.

Die verwendeten Thiocarbamoylchromen-Derivate der allgemeinen Formel VI werden gemäss DE-OS 2 801 353 nach bekannten Vorschriften durch Reaktion von o-Hydroxybenzaldehyd-Derivaten mit 2-Cyan-thioacetamid erhalten.

Die Ausgangsprodukte der allgemeinen Formel III sind in J. Chem. Soc. S. 1335, (1980), beschrieben.

Die Verbindungen der allgemeinen Formel IV werden vorzugsweise nicht isoliert, sondern in einer Art Eintopfverfahren direkt weiter umgesetzt mit Verbindungen der allgemeinen Formel V. Unter reaktiven Estergruppen werden Ester oder Halbester starker Mineralsäuren wie z.B. Halogenwasserstoffsäuren oder Schwefelsäure verstanden.

Bevorzugt werden die Bromide und Chloride.

Die Verbindungen V werden in Form ihrer Hydrobromide oder Hydrochloride eingesetzt. In diesem Fall verwendet man für die Alkylierungsreaktion 2 Moläquivalente der Base.

Die Umsetzung der Verbindungen der Formel VI erfolgt bevorzugt in Gegenwart einer Base wie Natriummethanolat oder Natriummethanolat in einem polaren Lösungsmittel.

Die Verbindungen der allgemeinen Formel I kristallisieren nach Filtration vom ausgefallenen Alkalihalogenid direkt aus dem Filtrat aus oder sie werden nach dem Einengen des Reaktionsgemisches und Verteilen des Rückstandes zwischen einem mit Wasser nicht mischbaren organischen Lösungsmittel wie z.B. Methylenchlorid oder Chloroform, und Wasser nach dem Einengen der organischen Phase und Kristallisieren aus einem geeigneten Lösungsmittel erhalten.

Die Salze werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Die Verbindungen der allgemeinen Formel I sind wertvolle Mittel zur Bekämpfung von gastralen und duodenalen Schleimhautläsionen.

Die gegenwärtige Therapie des peptischen Ulkus besteht entweder in einer Hemmung der Säuresekretion aus den Parietalzellen des Magens oder in einer Neutralisation der Magensäure bevor sie mit den ulcerierten Arealen in Berührung kommt.

Zur Anwendung kommen bisher insbesondere Pharmaka aus der Reihe der Anticholinergica oder Antacida. Erstere besitzen allgemein bekannte Nebenwirkungen, Antacida müssen häufig und in grossen Mengen appliziert werden.

Eine Wende in der Therapie war die Einführung des Histamin-$H_2$-Rezeptorenblockers Cimetidin; er erlaubte eine neue Form der antisekretorischen Behandlung. Cimetidin besitzt jedoch zentrale und andere Nebenwirkungen; besonders unangenehm sind die unter Langzeittherapie auftretenden Veränderungen der Magenschleimhaut, weil sie für Rezidiv-Ulcera nach Absetzen von Cimetidin verantwortlich gemacht werden.

[Int. J. Clinical Pharmacol., Therapy and Toxicol. 18/3 (1980), S. 140-43].

Die vorliegende Erfindung geht aus von dem Gedanken, dass die Unterstützung physiologischer Mechanismen der gastrointestinalen Mucoprotection, also die therapeutische Verhinderung von Läsionen des Schleimhautepithels jeder anderen, letzten Endes unphysiologischen Therapie ulcerativer Erkrankungen überlegen sein müsste. Die Hemmung oder gar Blockade der peptischen Sekretion als Ziel der Ulkustherapie hat nämlich beachtliche Nachteile für den Patienten. So häufen sich die Berichte über duodenale Mycosen, erosive, mykotische Gastritiden und systemische Candidiasis als Folge der verringerten gastralen zellulären Resistenz einerseits und des pH-Wert-Anstiegs durch Antisekretion andererseits. Die vorliegenden, erfindungsgemässen Verbindungen zeichnen sich durch eine ausgeprägte ulkusprotektive Wirkung bei völliger Abwesenheit von antisekretorischen Effekten aus. Ihre cytoprotektiven Effekte gegen chemische Noxen und Stressfaktoren lässt sie zur Therapie einer Reihe von Krankheitsbildern geeignet erscheinen. Solche sind beispielsweise das gastrische Ulcus, die Gastritis, das duodenale Ulcus und die ulcerative Colitis. Sie eignen sich auch für die Unterstützung bei der Therapie mit Antirheumatica, Steroiden oder Cytostatica.

Die Verbindungen dieser Erfindung können für alle üblichen Applikationsformen eingesetzt werden; die oralen Formen sind jedoch bevorzugt. Die Applikationsformen enthalten dabei die üblichen Excipienten, Füll- und Gleitstoffe und desintegrierende Zusätze. Derartige Zusätze sind z.B. Tartrat- und Citrat-Puffer, Ethanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol) für orale Applikation geeignete Zubereitungen können gewünschtenfalls zusätzliche Geschmacks- und/oder Süssstoffe enthalten.

Die bevorzugten Einzeldosierungen liegen zwischen 10 und 500 mg des entsprechenden aktiven Inhaltsstoffes.

Ein weiterer Gegenstand der Erfindung sind daher Arzneimittel, die dadurch gekennzeichnet sind, dass sie neben üblichen Füll- und Hilfsstoffen mindestens eine Verbindung gemäss der allgemeinen Formel I enthalten, sowie die Verwendung von Verbindungen gemäss der allgemeinen Formel I bei der Bekämpfung von gastralen und duodenalen Schleimhautläsionen. Die folgenden Beispiele sollen die Erfindung näher erläutern.

*Beispiel 1*

*4-(3-Dimethylaminopropylthio)-2-phenyl-5H-[1]-benzopyrano[2,3-d]pyrimidin* (1)

Eine Mischung von 11,7 g 2,3-Dihydro-2-phenyl--4-thioxo-[1]-benzopyrano[2,3-d]pyrimidin, 11,5 g Kaliumcarbonat und 300 ml Ethanol wird 30 Min. unter Rückfluss erhitzt. Dann tropft man innerhalb von 30 Min. eine Lösung von 6,3 g Dimethylaminopropylchlorid Hydrochlorid in 100 ml Ethanol hinzu und rückflussiert weitere 2 Stunden. Die Reaktionsmischung wird heiss filtriert, eingeengt und der Rückstand aus Isopropoanol kristallisiert. Man erhält 8,7 g farblose Kristalle, Fp. 94°C.

Das als Ausgangsprodukt verwendete 2,3-Dihydro-2-phenyl-4-thioxo [1]benzopyrano[2,3-d]--pyrimidin (28) wird wie folgt hergestellt:

10,0 g 2-Imino-3-thiocarbamoyl-(2H)-chromen, 5,2 g Benzaldehyd und 5 Tropfen Piperidin werden 6 Stunden in 1 l Benzol am Wasserabscheider gekocht. Nach dem Abkühlen wird das ausgefallene gelbe Reaktionsprodukt abgesaugt, mit etwas Benzol ausgewaschen und im Vakuum getrocknet. Ausbeute 11,0 g gelbe Kristalle, Fp. 220°C, zers.

In analoger Weise erhält man folgende Verbindungen:

*4-(2-Diethylaminoethylthio)-2-phenyl-5H-[1]benzopyrano[2,3-d]pyrimidin* (2)
Ausbeute 71%, Fp. 109°C
*4-(2-Morpholinoethylthio)-2-phenyl-5H-[1]benzopyrano[2,3-d]pyrimidin* (3)
Ausbeute 77%, Fp. 155°C

*Beispiel 2*

*2-(4-Chlorphenyl)-4-(2-piperidinoethylthio)-5H-[1]-benzopyrano[2,3-d]pyrimidin* (4)

Man löst 1,6 g Natrium in 500 ml abs. Ethanol, gibt 11,4 g 2-(4-Chlorphenyl)-2,3-dihydro-4-thioxo--benzopyrano[2,3-d]pyrimidin hinzu und erhitzt 30 Min. zum Sieden. Dann tropft man eine Lösung von 6,4 g N-(2-Chlorethyl)piperidin Hydrochlorid in 100 ml abs. Ethanol hinzu und rückflussiert 1 Stunde weiter. Man kühlt ab, saugt den ausgefallenen Niederschlag ab und wäscht gut mit Wasser aus. Schliesslich kristallisiert man aus Dimethylformamid. Man erhält 12,1 g gelbliche Kristalle vom Fp. 179°C.

In analoger Weise werden erhalten:

*2-(4-Chlorphenyl)-4-(3-dimethylaminopropylthio)--5H-[1]benzopyrano[2,3-d]pyrimidin* (5)
Ausbeute 57%, Fp. 118°C

*2-(4-Chlorphenyl)-4-(2-diethylaminoethylthio)-5H--[1]benzopyrano[2,3-d]pyrimidin* (6)
Ausbeute 59%, Fp. 116°C

*2-(4-Dimethylaminophenyl)-4-(2-morpholinoethylthio)-5H-[1]benzopyrano[2,3-d]pyrimidin* (7)
Ausbeute 93%, Fp. 181°C

*2-(4-Dimethylaminophenyl)-4-(3-dimethylaminopropylthio)-5H-[1]benzopyrano[2,3-d]pyrimidin*(8)
Ausbeute 60%, Fp. 131°C

*2-(4-Dimethylaminophenyl)-4-(2-piperidinoethylthio)-5H-[1]benzopyrano[2,3-d]pyrimidin* (9)
Ausbeute 87%, Fp. 162°C

*4-(3-Dimethylaminopropylthio)-2-(3,4-methylendioxyphenyl)-5H-[1]benzopyrano[2,3-d]pyrimidin* (12)
Ausbeute 66%, Fp. 125°C

*4-(2-Dimethylaminoethylthio)-2-(3,4-methylendioxyphenyl)-5H-[1]benzopyrano[2,3-d]pyrimidin*(13)
Ausbeute 70%, Fp. 167°C

*4-(2-Piperidinoethylthio)-2-(3,4,5-trimethoxyphenyl)-5H-[1]benzopyrano[2,3]pyrimidin* (14)
Ausbeute 84%, Fp. 168°C

*4-(2-Diethylaminoethylthio)-2-(4-methylphenyl)--5H-[1]benzopyrano[2,3-d]pyrimidin* (15)
Ausbeute 59%, Fp. 118°C

*4-(3-Dimethylaminopropylthio)-2-(4-methylphenyl)-5H-[1]benzopyrano[2,3-d]pyrimidin* (16)
Ausbeute 65%, Fp. 109°C

*4-(2-Morpholinoethylthio)-2-(4-methylphenyl-5H--[1]benzopyrano[2,3-d]pyrimidin* (17)
Ausbeute 87%, Fp. 178°C

*4-(3-Dimethylaminopropylthio)-2-(4-methoxyphenyl)-5H-[1]benzopyrano[2,3-d]pyrimidin* (18)
Ausbeute 70%, Fp. 100°C

*4-(2-Dimethylaminoethylthio)-7-methoxy-2-phenyl-5H-[1]benzopyrano[2,3-d]pyrimidin* (19)
Ausbeute 61%, Fp. 140°C

*7-Brom-4-(2-morpholinoethylthio)-2-phenyl-5H-[1]-benzopyrano[2,3-d]pyrimidin* (20)
Ausbeute 73%, Fp. 190°C

*9-Ethoxy-2-phenyl-4-(2-pyrrolidinoethylthio)-5H--[1]benzopyrano[2,3-d]pyrimidin* (21)
Ausbeute 52%, Fp. 150°C

*6,8-Dimethoxy-2-phenyl-4-(2-piperidinoethylthio)--5H-[1]benzopyrano[2,3-d]pyrimidin* (22)
Ausbeute 55%, Fp. 165°C

*4-(2-Aminoethylthio)-2-phenyl-5H-[1]benzopyrano-[2,3-d]pyrimidin* (23)
Ausbeute 70%, Fp. 152°C

*4-(2-Aminoethylthio)-7-methoxy-2-(4-methoxyphenyl)-5H-[1]benzopyrano[2,3-d]pyrimidin* (24)
Ausbeute 78%, Fp. 153°C

*4-(2-Aminoethylthio)-7-brom-2-(4-methoxyphenyl)-5H-[1]benzopyrano[2,3-d]pyrimidin* (25)
Ausbeute 60%, Fp. 196°C

*2-(4-Methylphenyl)-4-(3-morpholinopropylthio)--5H-[1]benzopyrano[2,3-d]pyrimidin* (26)
Ausbeute 73%, Fp. 139°C

*4-(3-Aminopropylthio)-2(4-methylphenyl)-5H-[1]-benzopyrano[2,3-d]pyrimidin* (27)
Ausbeute 32%, Fp. 131°C

*11-(2-Diethylaminoethylthio)-9-phenyl-12H--naphto[1',2':5,6]pyrano[2,3-d]pyrimidin* (27b)
Ausbeute 70%, Fp. 174°C

*4-(3-methylaminopropylthio)-2-(4-methylphenyl)--5H-[1]benzopyrano[2,3-d]pyrimidin* (27a)
Ausbeute 46%, Fp. 125-127°C

*4-(2-Diethylaminoethylthio)-2-(4-hydroxyphenyl)--5H-[1]benzopyrano[2,3-d]pyrimidin* (10)
Ausbeute 59%, Fp. 178°C

*2-(4-Hydroxyphenyl)-4-(2-morpholinoethylthio)--5H-[1]benzopyrano[2,3-d]pyrimidin* (11)
Ausbeute 47%, Fp. 225°C

*4-Hexylthio-2-(3,4-methylendioxyphenyl)-5H-[1]-benzopyrano[2,3-d]pyrimidin* (35)
Ausbeute 68%, Fp. 111°C

*4-(3-Dimethylaminopropylthio)-2-(3,4,5-trimethoxyphenyl)-5H-[1]benzopyrano[2,3-d]pyrimidin* (36)
Ausbeute 70%, Fp. 162°C

*4-(3-Dimethylaminopropylthio)-7,8-methylendioxy--2-(3,4-methylendioxyphenyl)-5H-[1]benzopyrano-[2,3-d]pyrimidin* (37)
Ausbeute 49%, Fp. 164°C

*4-(6-Dimethylaminohexylthio)-2-(3,4-methylendioxyphenyl)-5H-[1]benzopyrano[2,3-d]pyrimidin*(38)
Ausbeute 56%, Fp. 96°C

*6-(3-Dimethylaminopropylthio)-2,3-dihydro-8-(4--methylphenyl)-5H-furo[2',3':7,8][1]benzopyrano-[2,3-d]pyrimidin* (39)
Ausbeute 69%, Fp. 171°C.

Bei der Herstellung der Verbindungen (10) und (11) verwendet man für die Tautomerisierungsreaktion 3 Molequivalente Natrium und arbeitet den Ansatz wie folgt auf:

Man engt die Reaktionsmischung am Rotationsverdampfer ein, giesst den Rückstand in 300 ml Wasser und stellt mit verd. Salzsäure ein pH von 7 ein. Der hierbei ausgefallene Niederschlag wird abgesaugt und aus Ethanol kristallisiert.

Die zur Herstellung der genannten Verbindungen verwendeten 2-Aryl-2,3-dihydro-4-thioxo-benzopyrano(2,3-D)pyrimidine der allgemeinen Formel II werden gemäss Beispiel 1 durch Kondensation von 2-Imino-3-thiocarbamoyl-(2H)-chromenen der allgemeinen Formel VI mit geeigneten Aldehyden der allgemeinen Formel VII erhalten:

2-(4-Chlorphenyl)-2,3-dihydro-4-thioxo-[1]benzopyrano[2,3-d]pyrimidin
Ausbeute 61%, gelbe Kristalle, Fp. 246°C, zers.

2-(4-Dimethylaminophenyl)-2,3-dihydro-4-thioxo--[1]benzopyrano]2,3-d]pyrimidin
Ausbeute 63%, orangefarbene Kristalle, Fp. 218°C, zers.

2,3-Dihydro-2-(4-hydroxyphenyl)-4-thioxo-[1]-benzopyrano[2,3-d]pyrimidin
Ausbeute 80%, gelbe Kristalle, Fp. 248°C, zers.

2,3-Dihydro-2-(4-methylphenyl)-4-thioxo-[1]benzopyrano[2,3-d]pyrimidin
Ausbeute 52%, gelbe Kristalle , Fp. 205°C, zers.

2,3-Dihydro-2-(4-methoxyphenyl)-4-thioxo-[1]-benzopyrano[2,3-d]pyrimidin
Ausbeute 52%, gelbe Kristalle, Fp. 202°C, zers.

2,3-Dihydro-2-(3,4-methylendioxyphenyl)-4--thioxy-[1]benzopyrano[2,3-d]pyrimidin
Ausbeute 56%, gelbe Kristalle, Fp. 226°C, zers.
2,3-Dihydro-2-(3,4,5-trimethoxyphenyl)-4-thioxo--[1]benzopyrano[2,3-d]pyrimidin
Ausbeute 85%, gelbe Kristalle, Fp. 290°C, zers.
2,3-Dihydro-7-methoxy-2-phenyl-4-thioxo-[1]-benzopyrano[2,3-d]pyrimidin
Ausbeute 87%, gelbe Kristalle, Fp. 223°C, zers.
9,10-Dihydro-9-phenyl-11-thioxy-naphto-[1',2':5,6]pyrano[2,3-d]pyrimidin
Ausbeute 85%, 320°C, zers.
7-Brom-2,3-dihydro-2-phenyl-4-thioxo-[1]benzo-pyrano[2,3-d]pyrimidin
Ausbeute 80%, Fp. 330°C, zers.
9-Ethoxy-2,3-dihydro-2-phenyl-4-thioxo-[1]benzo-pyrano[2,3-d]pyrimidin
Ausbeute 77%, Fp. 215°C, zers.
2,3-Dihydro-6,8-dimethoxy-2-phenyl-4-thioxo--[1]benzopyrano[2,3-d]pyrimidin
Ausbeute 68%, Fp. 298°C, zers.
2,3-Dihydro-7-Methoxy-2-(4-methoxyphenyl)-4--thioxo-[1]benzopyrano[2,3-d]pyrimidin
Ausbeute 72%, Fp. 250°C, zers.
7-Brom-2,3-dihydro-2-(4-methoxyphenyl)-4--thioxo-[1]benzopyrano[2,3-d]pyrimidin
Ausbeute 75%, Fp. 235°C, zers.
2,3-Dihydro-7,8-methylendioxy-2-(3,4-methylendioxyphenyl)-4-thioxo-[1]benzopyrano[2,3-d]-pyrimidin
Ausbeute 93%, Fp. 270°C, zers.
2,3,7,8-Tetrahydro-8-(4-methylphenyl)-6-thioxo--furo[2',3':7,8][1]benzopyrano[2,3-d]pyrimidin
Ausbeute 80%, Fp. ab 210°C, zers.

*Beispiel 3*

*2-Phenyl-4-thioxo-3H,5H-[1]benzopyrano[2,3-d]-pyrimidin* (29)

Man löst 1,1 g Natrium in 300 ml abs. Ethanol, gibt 14,6 g 2,3-Dihydro-2-phenyl-4-thioxo-[1]-benzo-pyrano[2,3-d]pyrimidin hinzu und erhitzt 1 Stunde zum Sieden. Danach engt man die Reaktionsmischung am Rotationsverdampfer ein, giesst in 300 ml Wasser und säuert vorsichtig mit verd. Salzsäure an. Der ausgefallene Niederschlag wird abgesaugt und aus Dimethylformamid/Ethanol kristallisiert.

Ausbeute 80% gelbe Kristalle, Fp. 245°C, (zers.)

In analoger Weise werden erhalten:

*2-(4-Dimethylaminophenyl)-4-thioxo-3H,5H-[1]-benzopyrano[2,3-d]pyrimidin* (30)
Ausbeute 76%, gelbe Kristalle, Fp. 245°C, zers.
*2-(4-Methoxyphenyl)-4-thioxo-3H,5H-[1]benzo-pyrano[2,3-d]pyrimidin* (31)
Ausbeute 74%, gelbe Kristalle, Fp. 265°C, zers.
*2-(4-Chlorphenyl)-4-thioxo-3H,5H-[1]benzopyrano-[2,3-d]pyrimidin* (32)
Ausbeute 70%, gelbe Kristalle, Fp. 340°C, zers.
*4-Thioxo-2-(3,4,5-trimethoxyphenyl)-3H,5H-[1]-benzopyrano[2,3-d]pyrimidin* (33)
Ausbeute 79%, ockerfarbene Kristalle, Fp. 290°C, zers.
*2-(4-Hydroxyphenyl)-4-thioxo-3H,5H-[1]benzo-pyrano[2,3-d]pyrimidin* (34)
Ausbeute 65%, gelbliche Kristalle, Fp. 274°C, zers.

*Beispiel 4*

*4-(3-Dimethylaminopropylthio)-2-phenyl-5H-[1]-benzopyrano[2,3-d]pyrimidin*

8,1 g 4-Hydroxy-2-phenyl-[1]benzopyrano[2,3-d]-pyrimidin, 9,0 g Phosphorpentachlorid und 36 ml Phosphoroxychlorid werden 2 Stunden unter Rückfluss erhitzt. Danach wird das überschüssige Phosphoroxychlorid im Wasserstrahlvakuum abdestilliert und der Rückstand zwischen Chloroform und Wasser verteilt, wobei durch Zugabe von 10%iger Natronlauge ein pH Wert von ca. 6 eingestellt wird.

Die organische Phase wird abgetrennt, das Chloroform am Rotationsverdampfer entfernt und der Rückstand an Kieselgel mit Cyclohexan/Chloroform (1:1) als Elutionsmittel chromatographiert. Man erhält 4,0 g 4-Chlor-2-phenyl-[1]benzopyrano[2,3-d]-pyrimidin, Fp. 168-170°C in Form farbloser Kristalle.

0,05 g Natrium werden in 10 ml abs. Ethanol gelöst, und die Lösung mit 0,25 g 3-Dimethylamino-1--propanthiol versetzt.

Diese Lösung tropft man bei Rückflusstemperatur in eine Lösung von 0,6 g 4-Chlor-2-phenyl[1]benzo-pyrano[2,3-d]pyrimidin und hält danach 2 Stunden unter Rückfluss.

Nach dem Abkühlen wird filtriert, das Filtrat zur Trockene eingeengt und der Rückstand zwischen Chloroform und Wasser verteilt. Die organische Phase wird abgetrennt, das Chloroform abgezogen und der Rückstand aus Ethanol kristallisiert. Man erhält 0,4 g farblose Kristalle, Fp. 93°C.

In analoger Weise erhält man

*4-(4-Dimethylaminobutylthio)-2-(3,4-methylendioxyphenyl)-5H-[1]benzopyrano[2,3-d]pyrimidin*
Ausbeute 64%, Fp. 97-98°C.

Die folgenden Vergleichsversuche zeigen die neuartige Wirkungsweise der Verbindungen gemäss der allgemeinen Formel I im Vergleich mit Cimetidin.

*Pharmakologische Methoden*

1. *Indomethacin-Ulkus*

Versuchstiere waren männliche Ratten (Sprague Dawley SIV 50) mit einem Körpergewicht von 150-200 g. 24 Stunden vor Beginn des Versuches wurde den Tieren das Futter entzogen. Trinkwasser stand ad libitum zur Verfügung. Die Tiere wurden in 3 Gruppen zu je 10 Ratten eingeteilt. Zur Erzeugung der Ulcerationen wurde allen Tieren Indomethacin in einer Dosierung von 40 mg/kg i.g. appliziert. Gleichzeitig erhielten die Gruppen das Vehikel (Kontrollgruppe) die Substanzsuspension (Testgruppe) bzw. eine Vergleichssubstanz (Cimetidin 100/200 mg/kg). 5 Stunden nach der Applikation wurden die Tiere durch $CO_2$-Gas getötet, der Magen exstirpiert und entlang der grossen Kurvatur geöffnet.

Nach Messung des pH-Wertes wurde der Magen unter einer Stereolupe auf Ulcerationen untersucht. Die Bewertung der Ulcerationen und die Berechnung des Ulkusindex (UI) erfolgte nach der von Chaumontet et al. Arzneimittelforschung/Drug Research 28 (II) S. 2119-2121 (1978) beschriebenen Methode. Die Wirkung der Substanzen wurde als prozentuale Hemmung der durch Indomethacin hervorgerufenen

Ulcera im Vergleich zur unbehandelten Kontrollgruppe angegeben.

2. *Kälte-Stress-Ulcus*

Als Versuchstiere dienten männliche Ratten (Sprague Dawley SIV 57) mit einem Körpergewicht zwischen 110 und 150 g. 24 Stunden vor Versuchsbeginn wurde den Tieren das Futter entzogen; Trinkwasser stand ad libitum zur Verfügung.

Die Ratten wurden in 3 Gruppen zu je 10 Tieren eingeteilt, wobei einer Gruppe das Vehikel (0,8% Methocel), der zweiten Gruppe die Testsubstanz als Suspension in 0,8% Methocel und der dritten Gruppe ein Vergleichsstandard (Cimetidin 50 mg/kg) i.g. appliziert wurde. Unmittelbar nach Verabreichung der Substanzen wurden die Tiere mit Penthrane narkotisiert und mit Klebeband auf dem Rücken liegend auf einem Holzbrettchen fixiert.

So immobilisiert wurden die Tiere für 14 Stunden in einen auf +15°C temperierten, permanent beleuchteten Raum mit einer relativen Luftfeuchtigkeit von 50-60% gebracht.

Nach der genannten Expositionszeit wurden die Ratten mit $CO_2$ getötet, der Magen exstirpiert und entlang der grossen Kurvatur geöffnet.

Nach Messung des pH-Wertes wurde die Magenschleimhaut unter Stereolupenvergrösserung auf Ulcerationen untersucht. Die Bewertung der Ulcerationen und die Berechnung des Ulkusindex (UI) erfolgte wie bei Versuch 1, wobei die Wirkung der Substanzen als prozentuale Hemmung der Ulkusbildung im Vergleich zur unbehandelten Kontrollgruppe angegeben wurde.

3. *Untersuchung zur Hemmung der Magensäuresekretion nach Ghosh und Schild* (Brit. J. Pharmacol. 13 S. 54-61 (1958)

Als Versuchstiere dienten männliche Ratten (Sprague Dawley SIV 50) im Gewicht von 300-450 g. 24 Stunden vor Versuchsbeginn wurde das Futter entzogen, Wasser stand ad libitum zur Verfügung.

Die Tiere wurden mit 1,25 g/kg Urethan i.p. narkotisiert.

Die Trachea, die Vena jugularis, die Arteria carotis und der Oesophagus wurden freipräpariert, anschliessend wurde eine Pylorusfistel angelegt. Über eine Ösophagussonde wurde der Magen mit 1/4000 N NaOH bei einer Flussgeschwindigkeit von 3 ml/min perfundiert. Mit Hilfe der NaOH-Perfusion wurde der pH-Wert des Magensaftes auf annähernd pH 7 eingestellt und über eine Messelektrode kontinuierlich gemessen.

Über die Vena jugularis wurde nun die säurestimulierende Substanz (Carbachol 0,25 µg/kg/min bzw. Pentagastrin 1 µg/kg/min) infundiert. Bei Erreichen einer maximalen Sekretion (pH 3,5) konnte über einen Duodenalkatheter die Testsubstanz als Suspension in 0,8% Methocel verabreicht werden.

Eine Stunde nach Applikation der Testsubstanz wurde als sekretionshemmende Vergleichssubstanz Cimetidin 30 mg/kg i.d. gegeben. Der Versuch wurde abgeschlossen, nachdem der Ausgangs-pH-Wert von 7 wieder erreicht war.

*Untersuchung der akuten Toxizität*

*Methode:*

Die Bestimmung der akuten Toxizität wurde an männlichen Mäusen (NMRI) mit einem Körpergewicht von 20 bis 25 g durchgeführt. Alle Versuchstiere wurden 20 Stunden vor Versuchsbeginn nüchtern gesetzt. Wasser stand ad libitum zur Verfügung. Zu jeder Dosierungsgruppe gehörten 4 Tiere. Die Dosenfolge war logarithmisch. Die Prüfsubstanzen wurden intragastral als Suspension in 0,8%igem Methocel verabreicht. Das Applikationsvolumen betrug 20 ml/kg Körpergewicht. Die Tiere wurden insgesamt 7 Tage beobachtet.

Zur Schonung von Tieren wurden Dosierungen, die $LD_{50}$-Werten über 1600 mg/kg entsprachen, nicht berücksichtigt.

In der folgenden Tabelle I sind die Ergebnisse der Versuche wiedergegeben.

TABELLE I

| Substanz | Dosis mg/kg i.g. | Ulkusmodelle : Protektive Wirkung in % «Indometacin» | «Stress» | Antisekretion (Gosh + Schild-Modell) | LD50 (Maus) mg/kg i.g. (7-Tage-Werte) |
|---|---|---|---|---|---|
| Verbindung (1) | 125 | 88 | 70 | kein Effekt | >1600 |
| | 250 | 95 | 86 | kein Effekt | |
| Verbindung (16) | 125 | 83 | | kein Effekt | >1600 |
| | 250 | 99,5 | 72 | kein Effekt | |
| Verbindung (26) | 250 | 50 | | kein Effekt | >1600 |
| Verbindung (27a) | 250 | 94 | | kein Effekt | >1600 |
| Cimetidin | 5 i.v. | | | pH 7,9 nach 35 Min. | |
| | 100 | 80 | 53 | | |
| | 200 | 89 | 54 | | 2600 |
| | 3 × 100 | | 39 | | |

Die Ergebnisse der Vergleichsversuche zeigen, dass die Verbindungen der allgemeinen Formel I in beiden Ulkusmodellen eine hohe protektive Wirkung aufweisen, die überwiegend denjenigen des CIMETIDIN deutlich überlegen ist. Dabei zeigen jedoch die erfindungsgemässen Substanzen in keinem Fall einen antisekretorischen Effekt. Die Toxizität liegt bei allen untersuchten Verbindungen über 1600, d.h. in derselben Grössenordnung wie diejenige des CIMETIDINS.

Es handelt sich somit um einen bisher nicht bekannten, hoch wirksamen Typ ulkusprotektiver Substanzen ohne nennenswerte Nebenwirkungen.

**Patentansprüche für die Vertragsstaaten:**
BE, CH, DE, FR, GB, IT, LI, LU, NL, SE

1. 5H-[1]Benzopyrano-[2,3-d]pyrimidin-Derivate der allgemeinen Formel I

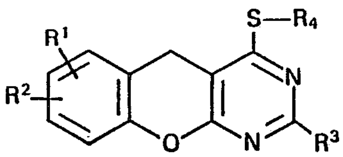

(I),

in welcher $R^1$ und $R^2$, die gleich oder verschieden sein können, ein Wasserstoff- oder Halogenatom, eine Hydroxylgruppe oder eine geradkettige oder verzweigte Alkoxygruppe mit bis zu 4 Kohlenstoffatomen, oder gemeinsam eine Alkylenoxy- oder eine Alkylendioxygruppe mit 1 bis 3 Kohlenstoffatomen oder einen ankondensierten Benzolring, $R^3$ einen unsubstituierten oder durch bis zu 3 Substituenten aus der Gruppe Halogenatome, Dialkylaminogruppen mit bis zu 4 Kohlenstoffatomen, Alkyl- oder Alkoxygruppen mit bis zu 4 Kohlenstoffatomen sowie bis zu 2 Alkylendioxygruppen mit bis zu 2 Kohlenstoffatomen substituierten Phenylrest, $R^4$ eine geradkettige oder verzweigte Alkylgruppe mit 1-8 Kohlenstoffatomen oder den Rest

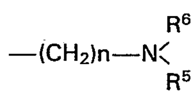

worin $R^5$ und $R^6$, die gleich oder verschieden sein können, ein Wasserstoffatom, eine geradkettige oder verzweigte Alkylgruppe mit bis zu 6 Kohlenstoffatomen oder gemeinsam mit dem Stickstoffatom eine Pyrrolidino-, Piperidino-, Morpholino- oder Piperazinogruppe bilden und n die Zahlen 2 bis 6 bedeuten,
sowie deren pharmakologisch verträgliche Salze mit organischen oder anorganischen Säuren.

2. Verbindungen gemäss der allgemeinen Formel I, Anspruch 1, in welcher $R^1$ und $R^2$ gleich oder verschieden sind und ein Wasserstoff- oder Bromatom, eine Hydroxyl-, Methoxy-, oder Ethoxygruppe oder gemeinsam eine Ethylenoxy- oder Methylendioxygruppe oder einen unsubstituierten, in 6,7-Position ankondensierten Benzolring, $R^3$ einen unsubstituierten oder durch ein Halogenatom, eine Dimethylamino-, eine Methyl-, eine Methylendioxygruppe oder einen durch bis zu 3 Methoxygruppen substituierten Phenylrest, $R^4$ einen Alkylrest mit 1-6 Kohlenstoffatomen oder einen Dialkylaminorest der Formel

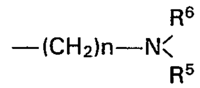

worin $R^5$ und $R^6$, die gleich oder verschieden sein können, ein Wasserstoffatom, einen Methyl- oder Ethylrest, oder zusammen mit dem Stickstoffatom, an das sie gebunden sind, eine Pyrrolidino-, Piperidino-, Morpholino- oder Piperazinogruppe und n die Zahlen 2 oder 3 bedeuten, sowie deren pharmakologisch verträgliche Salze.

3. 4-(3-Dimethylaminopropylthio)-2-phenyl-5H-[1]benzopyrano[2,3-d]pyrimidin.

4. Verfahren zur Herstellung von Verbindungen nach Anspruch 1 bis 3, dadurch gekennzeichnet, dass man entweder

a) eine Verbindung der allgemeinen Formel II,

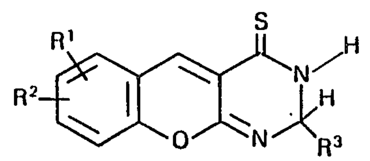

(II),

in welcher die Reste $R^1$, $R^2$ und $R^3$ die obengenannte Bedeutung haben,
in einem organischen Lösungsmittel mit einer Base tautomerisiert oder in dem man

b) eine Verbindung der allgemeinen Formel III

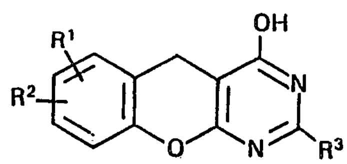

(III),

in welcher $R^1$, und $R^2$ und $R^3$ die obengenannte Bedeutung haben,
entweder

1) mit Phosphorpentasulfid in Pyridin umsetzt, und anschliessend die gemäss Verfahren a) oder b) erhaltene Verbindung der allgemeinen Formel IV

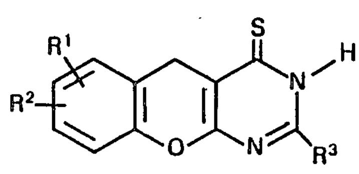

(IV),

in welcher die Reste $R^1$, $R^2$ und $R^3$ die obengenannte Bedeutung haben,
gegebenenfalls ohne Isolierung, mit einem Alkylierungsmittel der allgemeinen Formel V

$$X-R^4 \quad (V),$$

in welcher $R^4$ die obengenannte Bedeutung hat und X eine reaktive Estergruppe bedeutet, umsetzt oder

2) mit anorganischen Säurehalogeniden, bevorzugt mit Phosphoroxychlorid/Phosphorpentachlorid zu Verbindungen der allgemeinen Formel VI

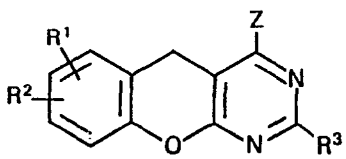

(VI),

in welcher die Reste $R^1$, $R^2$ und $R^3$ die oben genannte Bedeutung haben und Z für ein Chlor- bzw. Bromatom steht, umsetzt und diese mit einem Thioalkohol der allgemeinen Formel IIIb

$$HS—R^4 \qquad \text{(IIIb)}$$

in welcher $R^4$ die oben genannte Bedeutung hat, zur Reaktion bringt und die so erhaltenen Basen der allgemeinen Formel I gewünschtenfalls anschliessend in an sich bekannter Weise mit organischen oder anorganischen Säuren in deren pharmakologisch unbedenkliche Salze überführt.

5. Arzneimittel dadurch gekennzeichnet, dass es neben üblichen Füll- und Hilfsstoffen mindestens eine Verbindung gemäss Anspruch 1 bis 3 enthält.

**Patentansprüche für den Verstragsstaat:** AT

1. Verfahren zur Herstellung von Verbindungen gemäss der allgemeinen Formel I

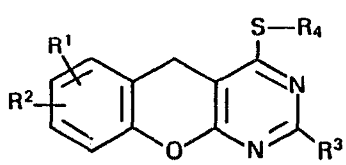

(I),

in welcher $R^1$ und $R^2$, die gleich oder verschieden sein können, ein Wasserstoff- oder Halogenatom, eine Hydroxylgruppe oder eine geradkettige oder verzweigte Alkoxygruppe mit bis zu 4 Kohlenstoffatomen, oder gemeinsam eine Alkylenoxy- oder eine Alkylendioxygruppe mit 1 bis 3 Kohlenstoffatomen oder einen ankondensierten Benzolring, $R^3$ einen unsubstituierten oder durch bis zu 3 Substituenten aus der Gruppe Halogenatome, Dialkylaminogruppen mit bis zu 4 Kohlenstoffatomen, Alkyl- oder Alkoxygruppen mit bis zu 4 Kohlenstoffatomen sowie bis zu 2 Alkylendioxygruppen mit bis zu 2 Kohlenstoffatomen substituierten Phenylrest, $R^4$ eine geradkettige oder verzweigte Alkylgruppe mit 1-8 Kohlenstoffatomen oder den Rest

$$—(CH_2)n—N\begin{matrix} R^6 \\ R^5 \end{matrix}$$

worin $R^5$ und $R^6$, die gleich oder verschieden sein können, ein Wasserstoffatom, eine geradkettige oder verzweigte Alkylgruppe mit bis zu 6 Kohlenstoffatomen oder gemeinsam mit dem Stickstoffatom eine Pyrrolidino-, Piperidino-, Morpholino- oder Piperazinogruppe bilden und n die Zahlen 2 bis 6 bedeuten, sowie von deren pharmakologisch verträglichen Salzen mit organischen oder anorganischen Säuren, dadurch gekennzeichnet, dass man entweder

a) eine Verbindung der allgemeinen Formel II,

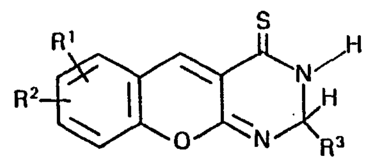

(II),

in welcher die Reste $R^1$, $R^2$ und $R^3$ die obengenannte Bedeutung haben, in einem organischen Lösungsmittel mit einer Base tautomerisiert oder in dem man

b) eine Verbindung der allgemeinen Formel III

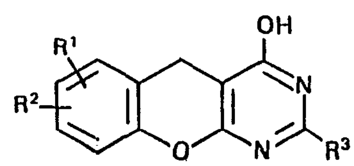

(III),

in welcher $R^1$ und $R^2$ und $R^3$ die obengenannte Bedeutung haben, entweder

1) mit Phosphorpentasulfid in Pyridin umsetzt, und anschliessend die gemäss Verfahren a) oder b) erhaltene Verbindung der allgemeinen Formel IV

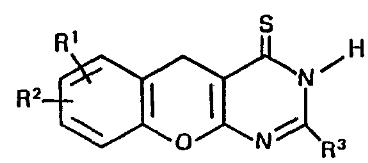

(IV),

in welcher die Reste $R^1$, $R^2$ und $R^3$ die obengenannte Bedeutung haben, gegebenenfalls ohne Isolierung, mit einem Alkylierungsmittel der allgemeinen Formel V

$$X—R^4 \qquad \text{(V)},$$

in welcher $R^4$ die obengenannte Bedeutung hat und X eine reaktive Estergruppe bedeutet, umsetzt oder

2) mit anorganischen Säurehalogeniden, bevorzugt mit Phosphoroxychlorid/Phosphorpentachlorid zu Verbindungen der allgemeinen Formel VI

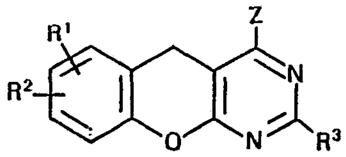

(VI),

in welcher die Reste $R^1$, $R^2$ und $R^3$ die oben genannte Bedeutung haben und Z für ein Chlor- bzw. Bromatom steht,
umsetzt und diese mit einem Thioalkohol der allgemeinen Formel IIIb

$HS-R^4$ (IIIb)

in welcher $R^4$ die oben genannte Bedeutung hat, zur Reaktion bringt und die so erhaltenen Basen der allgemeinen Formel I gewünschtenfalls anschliessend in an sich bekannter Weise mit organischen oder anorganischen Säuren in deren pharmakologisch unbedenkliche Salze überführt.

2. Verfahren zu Herstellung von Verbindungen gemäss der allgemeinen Formel I gemäss Anspruch 1, in welcher $R^1$ und $R^2$ gleich oder verschieden sind und ein Wasserstoff- oder Bromatom, eine Hydroxyl-, Methoxy-, oder Ethoxygruppe oder gemeinsam eine Ethylenoxy- oder Methylendioxygruppe oder einen unsubstituierten, in 6,7-Position ankondensierten Benzolring, $R^3$ einen unsubstituierten oder durch ein Halogenatom, eine Dimethylamino-, eine Methyl-, eine Methylendioxygruppe oder einen durch bis zu 3 Methoxygruppen substituierten Phenylrest, $R^4$ einen Alkylrest mit 1-6 Kohlenstoffatomen oder einen Dialkylaminorest der Formel

$R^6$
$-(CH_2)n-N\langle$
$R^5$

worin $R^5$ und $R^6$, die gleich oder verschieden sein können, ein Wasserstoffatom, einen Methyl- oder Ethylrest, oder zusammen mit dem Stickstoffatom, an das sie gebunden sind, eine Pyrrolidino-, Piperidino-, Morpholino- oder Piperazinogruppe und n die Zahlen 2 oder 3 bedeuten, sowie deren pharmakologisch verträglichen Salzen.

3. Verfahren gemäss Anspruch 1 oder 2 zur Herstellung von 4-(3-Dimethylaminopropylthio)-2-phenyl-5H-÷1]benzopyrano[2,3-d]pyrimidin.

4. Verwendung der nach Anspruch 1-3 hergestellten Verbindungen zur Herstellung von Arzneimitteln zur Bekämpfung von gastralen und duodenalen Schleimhautläsionen.

**Claims for the Contracting States:**
BE, CH, DE, FR, GB, IT, LI, LU, NL, SE,

1. 5H-[1]-Benzopyro[2,3-d]pyrimidine derivatives of the general formula I

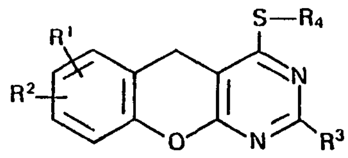

(I),

in which $R^1$ and $R^2$, which can be the same or different, signify a hydrogen or halogen atom, a hydroxyl group or a straight-chained or branched alkoxy group with up to 4 carbon atoms, or together an alkyleneoxy or an alkylenedioxy group with 1 to 3 carbon atoms or a condensed benzene ring, $R^3$ a phenyl radical, unsubstituted or substituted by up to 3 substituents from the group halogen atoms, dialkylamino groups with up to 4 carbon atoms, alkyl or alkoxy groups with up to 4 carbon atoms, es well as up to 2 alkylenedioxy groups up to 2 carbon atoms, $R^4$ a straight-chained or branched alkyl group with 1 - 8 carbon atoms or the radical

$R^6$
$-(CH_2)n-N\langle$
$R^5$

wherein $R^5$ and $R^6$, which can be the same or different, signify a hydrogen atom, a straight-chained or branched alkyl group with up to 6 carbon atoms or, together with the nitrogen atom, a pyrrolidino, piperidino, morpholino or piperazino group and n signifies the numbers 2 to 6, as well as their pharmacologically acceptable salts with organic or inorganic acids.

2. Compounds according to general formula I, claim 1, in which $R^1$ and $R^2$ are the same or different and signify a hydrogen or bromine atom, a hydroxyl, methoxy or ethoxy group or together an ethyleneoxy or methylenedioxy group or an unsubstituted benzene ring condensed on in the 6,7-position, $R^3$ a phenyl radical, unsubstituted or substituted by a halogen atom, a dimethylamino, a methyl, a methylenedioxy group or by up to 3 methoxy groups, $R^4$ an alkyl radical with 1 - 6 carbon atoms or a dialkylamino radical of the formula

$R^6$
$-(CH_2)n-N\langle$
$R^5$

wherein $R^5$ and $R^6$, which can be the same or different, signify a hydrogen atom, a methyl or ethyl radical or, together with the nitrogen atom to which they are attached, a pyrrolidino, piperidino, morpholino or piperazino group and n the numbers 2 or 3, as well as their pharmacologically acceptable salts.

3. 4-(3-Dimethylaminopropylthio)-2-phenyl-5H-[1]benzopyrano[2,3-d]pyrimidine.

4. Process for the preparation of compounds according to claim 1 to 3, characterised in that one either

a) tautomerises a compound of the general formula II

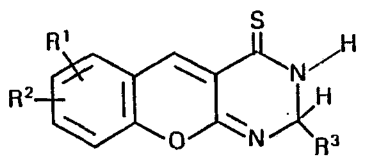

(II),

in which the radicals $R^1$, $R^2$ and $R^3$ have the above mentioned meaning, in an organic solvent with a base or in that one

b) reacts a compound of the general formula III

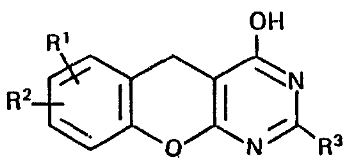

(III),

in which $R^1$, $R^2$ and $R^3$ have the above-mentioned meaning either

1) with phosphorus pentasulphide in pyridine and subsequently reacts the compound obtained according to process a) or b) of the general formula IV

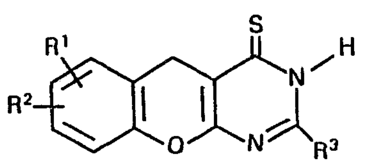

(IV),

in which the radical $R^1$, $R^2$ and $R^3$ have the above-mentioned meaning, optionally without isolation, with an alkylation agent of the general formula V

$$X—R^4 \qquad (V)$$

in which $R^4$ has the above-mentioned meaning and X signifies a reactive ester group or

2) with inorganic acid halides, preferably with phosphorus oxychloride/phosphorus pentachloride, to compounds of the general formula VI

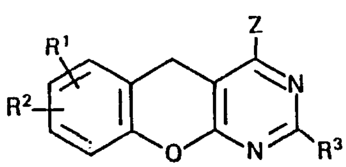

(VI),

in which the radicals $R^1$, $R^2$ and $R^3$ have the above-mentioned meaning and Z stands for a chlorine or bromine atom, and brings these to reaction with a thioalcohol of the general formula IIIb

$$HS—R^4 \qquad (IIIb)$$

in which $R^4$ has the above-mentioned meaning, and, if desired, subsequently converts the bases thus obtained of the general formula I in per se known manner with organic or inorganic acids into their pharmacologically acceptable salts.

5. Medicaments, characterised in that, besides the usual filler and adjuvant materials, it contains at least one compound according to claim 1 to 3.

**Claims for the Contracting State:** AT

1. Process for the preparation of compounds according to the general formula I

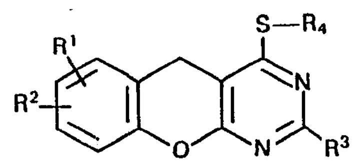

(I),

in which $R^1$ and $R^2$, which can be the same or different, signify a hydrogen or halogen atom, a hydroxyl group or a straight-chained or branched alkoxy group with up to 4 carbon atoms, or together an alkyleneoxy or an alkylenedioxy group with 1 to 3 carbon atoms or a condensed benzene ring, $R^3$ a phenyl radical, unsubstituted or substituted by up to 3 substituents from the group halogen atoms, dialkylamino groups with up to 4 carbon atoms, alkyl or alkoxy groups with up to 4 carbon atoms, es well as up to 2 alkylenedioxy groups up to 2 carbon atoms, $R^4$ a straight-chained or branched alkyl group with 1 - 8 carbon atoms or the radical

$$—(CH_2)n—N\begin{matrix} R^6 \\ R^5 \end{matrix}$$

wherein $R^5$ and $R^6$, which can be the same or different, signify a hydrogen atom, a straight-chained or branched alkyl group with up to 6 carbon atoms or, together with the nitrogen atom, a pyrrolidino, piperidino, morpholino or piperazino group and n signifies the numbers 2 to 6, as well as their pharmacologically acceptable salts with organic or inorganic acids, characterised in that one either

a) tautomerises a compound of the general formula II

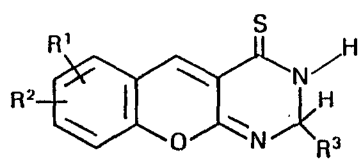

(II),

in which the radicals $R^1$, $R^2$ and $R^3$ have the above mentioned meaning, in an organic solvent with a base or in that one

b) reacts a compound of the general formula III

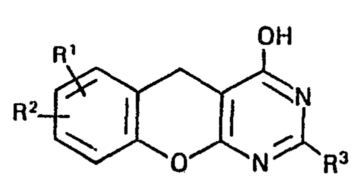

(III),

in which $R^1$, $R^2$ and $R^3$ have the above-mentioned meaning either

1) with phosphorus pentasulphide in pyridine and subsequently reacts the compound obtained according to process a) or b) of the general formula IV

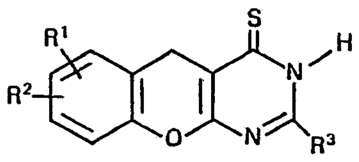

(IV),

in which the radicals $R^1$, $R^2$ and $R^3$ have the above-mentioned meaning, optionally without isolation, with an alkylation agent of the general formula V

$$X—R^4 \qquad (V)$$

in which $R^4$ has the above-mentioned meaning and X signifies a reactive ester group or

2) with inorganic acid halides, preferably with phosphorus oxychloride/phosphorus pentachloride, to compounds of the general formula VI

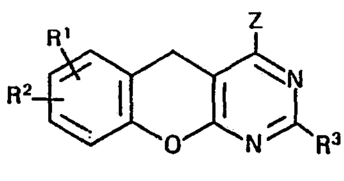

(VI),

in which the radicals $R^1$, $R^2$ and $R^3$ have the above-mentioned meaning and Z stands for a chlorine or bromine atom, and brings these to reaction with a thioalcohol of the general formula IIIb

$$HS—R^4 \qquad (IIIb)$$

in which $R^4$ has the above-mentioned meaning, and, if desired, subsequently converts the bases thus obtained of the general formula I in per se known manner with organic or inorganic acids into their pharmacologically acceptable salts.

2. Process for the preparation of compounds according to general formula I according to claim 1, in which $R^1$ and $R^2$ are the same or different and signify a hydrogen or bromine atom, a hydroxyl, methoxy or ethoxy group or together an ethyleneoxy or methylenedioxy group or an unsubstituted benzene ring condensed on in the 6,7-position, $R^3$ a phenyl radical, unsubstituted or substituted by a halogen atom, a dimethylamino, a methyl, a methylenedioxy group or a phenyl radical substituted by up to 3 methoxy groups, $R^4$ an alkyl radical with 1 - 6 carbon atoms or a dialkylamino radical of the formula

$$—(CH_2)n—N\begin{matrix} R^6 \\ R^5 \end{matrix}$$

wherein $R^5$ and $R^6$, which can be the same or different, signify a hydrogen atom, a methyl or ethyl radical or, together with the nitrogen atom to which they are attached, a pyrrolidino, piperidino, morpholino or piperazino group and n the numbers 2 or 3, as well as their pharmacologically acceptable salts.

3. Process according to claim 1 or 2 for the preparation of 4-(3-dimethylaminopropylthio)-2-phenyl-5H-[1]benzopyrano[2,3-d]pyrimidine.

4. Use of the compounds prepared according to claims 1 to 3 for the manufacture of pharmaceuticals useful for treating gastral and duodenal lesions of the mucal membranes.

**Revendications pour les Etats contractants:**
BE, CH, DE, FR, GB, IT, LI, LU, NL, SE,

1. Dérivés de la 5H-[1]benzopyrano[2,3-d]pyrimidine répondant à la formule générale I:

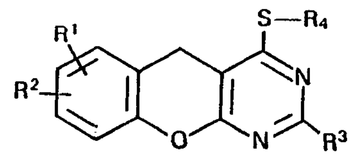

(I),

dans laquelle: $R^1$ et $R^2$, qui peuvent être identiques ou différents, représentent un atome d'hydxrogène ou d'halogène, un groupe hydroxy ou un groupe alkoxy à chaîne linéaire ou ramifiée contenant jusqu'à 4 atomes de carbone, ou bien forment ensemble un groupe alkylénoxy ou alkylènedioxy comportant de 1 à 3 atomes de carbone ou un noyau benzénique condensé sur le noyau; $R^3$ est constitué par un radical phényle non substitué ou substitué par au plus trois substituants appartenant au groupe des halogènes, des radicaux dialkylamines contenant jusqu'à 4 atomes de carbone, des radicaux alkyles ou alkoxy comprenant jusqu'à 4 atomes de carbone ainsi que par deux groupes alkylènedioxy comprenant au plus 2 atomes de carbone; $R^4$ est constitué par un groupe alkyle à chaîne linéaire ou ramifiée comprenant 1-8 atomes de carbone, ou le radical

$$—(CH_2)n—N\begin{matrix} R^6 \\ R^5 \end{matrix}$$

dans lequel $R^5$ et $R^6$, qui peuvent être identiques ou différents, représentent un atome d'hydrogène, un groupe alkyle à chaîne linéaire ou ramifiée comprenant au plus 6 atomes de carbone, ou constituent conjointement avec l'atome d'azote, un groupe pyrrolidino, pipéridino, morpholino ou pipérazino; et n est compris entre 2 et 6 bornes incluses; ainsi que leurs sels avec des acides organiques ou minéraux acceptables du point de vue pharmacologique.

2. Composés ayant la formule générale I selon la revendication 1, dans laquelle $R^1$ et $R^2$, sont identiques ou différents et représentent un atome d'hydrogène ou de brome, un groupe hydroxy, méthoxy ou éthoxy ou forment ensemble un groupe éthylénoxy ou méthylènedioxy ou un noyau benzénique non substitué, condensé en position 6, 7; $R^3$ est un radical phényle non substitué ou substitué par un atome d'halogène, un radical diméthylamino, un radical

méthyle, un radical méthylènedioxy ou par jusqu'à 3 radicaux méthoxy; $R^4$ un radical alkyle comprenant 1-6 atomes de carbone ou un radical dialkylamino répondant à la formule:

$$-(CH_2)n-N\langle^{R^6}_{R^5}$$

dans laquelle $R^5$ et $R^6$, qui peuvent être identiques ou différents, représentent un atome d'hydrogène, un radical méthyle ou éthyle ou constituent, conjointement avec l'atome d'azote auquel ils sont liés, un groupe pyrrolidino, pipéridino, morpholino ou pipérazino; et n est égal à 2 ou 3, ainsi que leurs sels acceptables du point de vue pharmacologique.

3. 4-(3-diméthylaminopropylthio)-2-phényl-5H-[1]benzopyrano[2,3-d]pyrimidine.

4. Procédé pour la préparation de composés selon les revendications 1 à 3, caractérisé en ce que, ou bien

a) on tautomérise le composé de formule générale II:

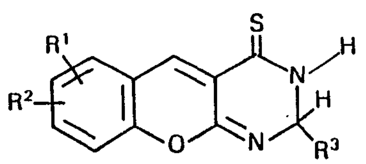

(II),

dans laquelle les radicaux $R^1$, $R^2$ et $R^3$ ont la signification ci-dessus mentionnée,
dans un solvant organique avec une base,

b) on transforme un composé de formule générale III:

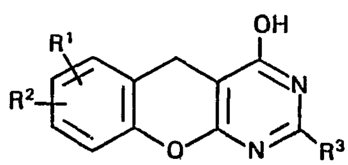

(III),

dans laquelle $R^1$ et $R^2$ et $R^3$ ont la signification ci-dessus mentionnée,
soit:

1) en pyridine par réaction avec du pentasulfure de phosphore et, ensuite, on transforme le composé obtenu d'après le procédé a) ou b), présentant la formule générale IV

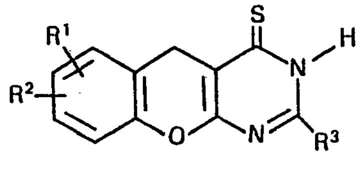

(IV),

dans laquelle les radicaux $R^1$, $R^2$ et $R^3$ ont la signification ci-dessus mentionnée,
le cas échéant sans isolement, par réaction avec un produit d'alkylation selon la formule générale V:

$$X-R^4 \qquad (V)$$

dans laquelle $R^4$ a la signification ci-dessus mentionnée et X représente un groupe ester réactif;
soit

2) en composés répondant à la formule générale VI:

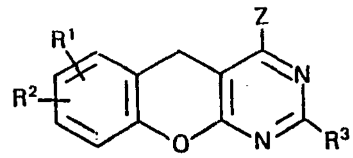

(VI),

dans laquelle les radicaux $R^1$, $R^2$ et $R^3$ ont la signification ci-dessus mentionnée; et Z correspond à un atome de chlore ou de brome, par réaction avec des halogénures d'acides minéraux, de préférence avec le mélange oxychlorure de phosphore/pentachlorure de phosphore et on provoque ensuite la réaction de ces composés avec un thio-alcool de formule générale IIIb:

$$HS-R^4 \qquad (IIIb)$$

dans laquelle $R^4$ a la signification ci-dessus indiquée, les bases répondant à la formule générale I ainsi obtenues, étant ensuite réduites à volonté, de manière connue en soi, avec des acides organiques ou minéraux, en sels inertes sous l'angle pharmacologique.

5. Médicament caractérisé en ce qu'il contient, en plus des charges et des adjuvants usuels, un composé selon les revendication 1 à 3.

**Revendications pour l'Etat contractant:** AT

1. Procédé de préparation de composés répondant à la formule générale I:

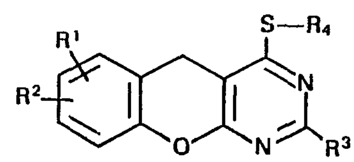

(I),

dans laquelle: $R^1$ et $R^2$, qui peuvent être identiques ou différents, représentent un atome d'hydxrogène ou d'halogène, un groupe hydroxy ou un groupe alkoxy à chaîne linéaire ou ramifiée contenant jusqu'à 4 atomes de carbone, ou bien forment ensemble un groupe alkylénoxy ou alkylènedioxy comportant de 1 à 3 atomes de carbone ou un noyau benzénique condensé sur le noyau; $R^3$ est constitué par un radical phényle non substitué ou substitué par au plus trois substituants appartenant au groupe des halogènes, des radicaux dialkylamines contenant jusqu'à 4 atomes de carbone, des radicaux alkyles ou alkoxy comprenant jusqu'à 4 atomes de carbone ainsi que par deux groupes alkylènedioxy comprenant au plus 2 atomes de carbone; $R^4$ est constitué par un groupe alkyle à chaîne linéaire ou ramifiée comprenant 1-8 atomes de carbone, ou le radical

$$-(CH_2)n-N\langle^{R^6}_{R^5}$$

dans lequel $R^5$ et $R^6$, qui peuvent être identiques ou différents, représentent un atome d'hydrogène, un groupe alkyle à chaîne linéaire ou ramifiée comprenant au plus 6 atomes de carbone, ou constituent conjointement avec l'atome d'azote, un groupe pyrrolidino, pipéridino, morpholino ou pipérazino; et n est compris entre 2 et 6 bornes incluses; ainsi que leurs sels avec des acides organiques ou minéraux acceptables du point de vue pharmacologique, caractérisé en ce que ou bien:

a) on tautomérise le composé de formule générale II:

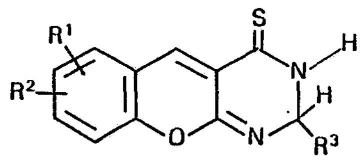

(II),

dans laquelle les radicaux $R^1$, $R^2$ et $R^3$ ont la signification ci-dessus mentionnée,
dans un solvant organique avec une base,

b) on transforme un composé de formule générale III:

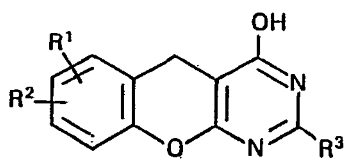

(III),

dans laquelle $R^1$ et $R^2$ et $R^3$ ont la signification ci-dessus mentionnée,
soit:
1) en pyridine par réaction avec du pentasulfure de phosphore et, ensuite, on transforme le composé obtenu d'après le procédé a) ou b), présentant la formule générale IV

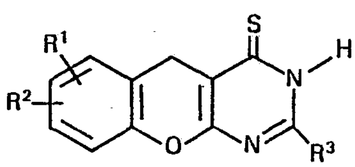

(IV),

dans laquelle les radicaux $R^1$, $R^2$ et $R^3$ ont la signification ci-dessus mentionnée,
le cas échéant sans isolement, par réaction avec un produit d'alkylation selon la formule générale V:

$$X-R^4 \qquad (V)$$

dans laquelle $R^4$ a la signification ci-dessus mentionnée et X représente un groupe ester réactif;
soit

2) en composés répondant à la formule générale VI:

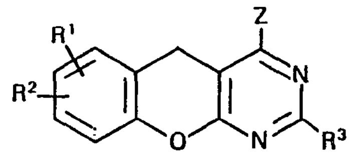

(VI),

dans laquelle les radicaux $R^1$, $R^2$ et $R^3$ ont la signification ci-dessus mentionnée; et Z correspond à un atome de chlore ou de brome, par réaction avec des halogénures d'acides minéraux, de préférence avec le mélange oxychlorure de phosphore/pentachlorure de phosphore et on provoque ensuite la réaction de ces composés avec un thio-alcool de formule générale IIIb:

$$HS-R^4 \qquad (IIIb)$$

dans laquelle $R^4$ a la signification ci-dessus indiquée, les bases répondant à la formule générale I ainsi obtenues, étant ensuite réduites à volonté, de manière connue en soi, avec des acides organiques ou minéraux, en sels inertes sous l'angle pharmacologique.

2. Procédé pour la préparation de composés ayant la formule générale I selon la revendication 1, dans laquelle: $R^1$ et $R^2$, sont identiques ou différents et représentent un atome d'hydrogène ou de brome, un groupe hydroxy, méthoxy ou éthoxy ou forment ensemble un groupe éthylénoxy ou méthylènedioxy ou un noyau de benzène non substitué, condensé en position 6, 7; $R^3$ est un radical phényle non substitué ou substitué par un atome d'halogène, un radical diméthylamino, un radical méthyle, un radical méthylènedioxy ou par jusqu'à 3 radicaux méthoxy; $R^4$ un radical alkyle comprenant 1-6 atomes de carbone ou un radical dialkylamino répondant à la formule:

$$-(CH_2)n-N\begin{matrix}R^6\\R^5\end{matrix}$$

dans laquelle $R^5$ et $R^6$, qui peuvent être identiques ou différents, représentent un atome d'hydrogène, un radical méthyle ou éthyle ou constituent, conjointement avec l'atome d'azote auquel ils sont liés, un groupe pyrrolidino, pipéridino, morpholino ou pipérazino; et n est égal à 2 ou 3, ainsi que leurs sels acceptables du point de vue pharmacologique.

3. Procédé selon la revendication 1 ou 2 pour la préparation de 4-(3-diméthylaminopropylthio)-2-phényl-5H-[1]benzopyrano[2,3-d]pyrimidine.

4. Application des composés préparés selon les revendications 1 à 3, à la fabrication de médicaments destinés à combattre les lésions des muqueuses stomacales et duodénales.